# EUROPÄISCHE PATENTSCHRIFT

(11) **EP 0 748 332 B1**
(45) Veröffentlichungstag und Bekanntmachung des Hinweises auf die Patenterteilung: **10.11.1999**
(21) Anmeldenummer: 95943161.0
(22) Anmeldetag: 28.12.1995
(51) Int. Cl.: C07J 41/00, A61K 9/127, A61K 48/00, C07J 9/00

(54) **NEUES CHOLESTEROLDERIVAT FÜR DEN LIPOSOMALEN GENTRANSFER**
NEW CHOLESTEROL DERIVATIVE FOR LIPOSOMAL GENE TRANSFER
NOUVEAU DERIVE DE CHOLESTEROL POUR LE TRANSFERT GENIQUE LIPOSOMAL

(30) Priorität: 28.12.1994 DE 4446937
(43) Veröffentlichungstag der Anmeldung: 18.12.1996
(73) Patentinhaber: MAX-DELBRÜCK-CENTRUM FÜR MOLEKULARE MEDIZIN, D-13125 Berlin (DE)
(72) Erfinder: RESZKA, Regina, D-16341 Schwanebeck (DE)
(74) Vertreter: Baumbach, Friedrich, Dr.
(86) Internationale Anmeldenummer: DE9501879
(87) Internationale Veröffentlichungsnummer: WO9620208

(56) Entgegenhaltungen:
- US-A- 5 283 185
- BIOCHEMICAL AND BIOPHYSICAL RESEARCH COMMUNICATIONS, Bd. 179, Nr. 1, 30.August 1991, ORLANDO, FL US, Seiten 280-285, XP000572654 X. GAO & L. HUANG: "A novel cationic liposome reagent for efficient transfection of mammalian cells" in der Anmeldung erwähnt
- JOURNAL OF LIPOSOME RESEARCH, Bd. 4, Nr. 1, Januar 1994, NEW YORK US, Seiten 289-299, XP000450213 A. SINGHAL & L. HUANG: "Direct gene transfer by liposomes"

## Beschreibung

Die Erfindung betrifft ein neues Cholesterolderivat für den liposomalen Gentransfer. Anwendungsgebiete der Erfindung sind die Medizin und die Gentechnik.

Kationische Liposomen sind effektive nichtvirale Transfektionsreagentien für tierische Zellen in vitro (P. Felgner, G. Ringold, Nature 337/1989/, 387-388). Das erste Reagenz dieser Art, DOTMA (N-[1-(2,3-dioleyloxy)propyl]-N,N,N-trimethylammoniumchlorid), ist nach Mischung mit einer äquimolaren Menge von DOPE (Dioleylphosphatidylethanolamin)in der Lage, eine Reihe von Säugerzellen in vitro und in vivo zu transfizieren.

Die Synthese von DOTMA verläuft über viele Stufen mit einer verhältnimäßig geringen Ausbeute. Das handelsübliche Mittel, Lipofektin, welches DOTMA und DOPE enthält, ist darüber hinaus relativ teuer. Andere kationische Liposomreagentien mit kommerziell zugänglichen kationischen Amphiphilen haben sich als relativ toxisch gegenüber den behandelten Zellen erwiesen (Pinnaduwage et al, Biochim. Biophys. Acta 285/1989/, 33-37).

X. Gao und L. Huang (Biochem. Biophys. Res. Comm. 179/1991/, 280-285) haben das kationische Cholesterolderivat 3β[N-(N',N'-Dimethylaminoethan)-carbamoyl]cholesterol (DC-Chol) beschrieben. Es kann in einer Stufe hergestellt werden, Liposomen mit diesem Lipid transfizieren effizienter und sind weniger toxisch gegenüber den behandelten Zellen als das Lipofektin-Reagenz.

Der Erfindung liegt die Aufgabe zugrunde, ein neues kationisches Lipid zu finden, das bei mit DC-Chol vergleichbarer Transfektionsfähigkeit eine geringere Toxizität aufweist und damit insbesondere für eine in vivo-Anwendung geeignet ist.

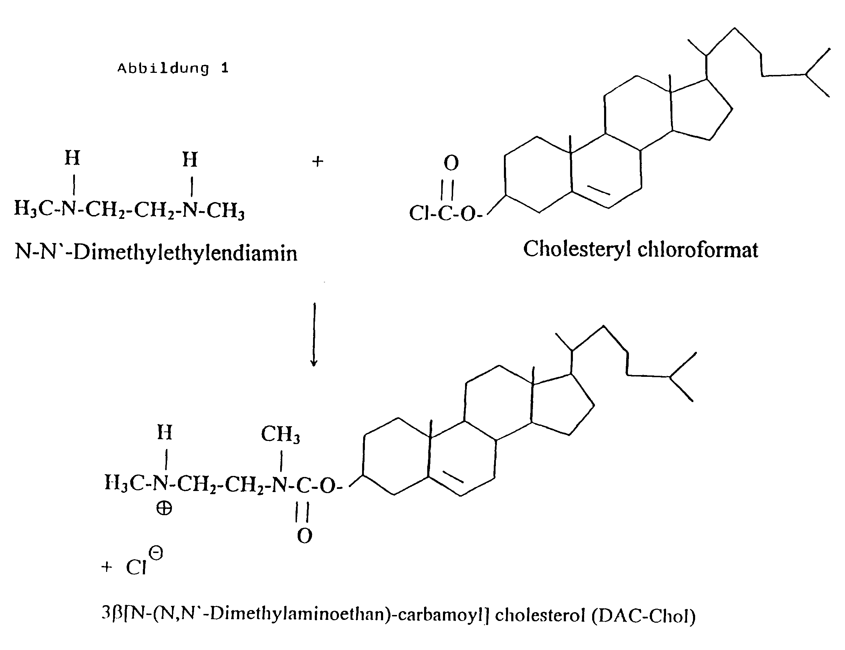

Die Aufgabe wird gemäß den Ansprüchen 1 und 2 gelöst, das Herstellungsverfahren ist in Abbildung 1 formelmäßig dargestellt.

Das neue Mittel unter Einsatz von DAC-Chol hat gegenüber den bisher verwendeten Mitteln den Vorteil, bei hochsensiblen Zellen nicht toxisch zu sein und sowohl in vitro als auch in vivo zu erfolgreichen Transfektionen zu führen. Die Anwendung des Mittels wird an Giablastoma-Zellen der Ratte, die sonst nur mit geringer Effektivität transfiziert werden können, gezeigt. Es werden Transfektionsraten erreicht, die im Vergleich zur Calciumphosphat-Präzipitationstechnik (CPPT) bis zu 10fach höher sind. Dieser Befund dürfte auf ein kompaktere Formation der DNA, einen besseren Liposom-Zellkontakt über die positiven Ladungen der Vesikel und auf die höhere Stabilität in Hinsicht auf die DNA abbauenden Enzyme zurückzuführen sein.

Dadurch kann es vorteilhaft für den direkten liposomalen Gentransfer gemäß Anspruch 4 eingesetzt werden. So lassen sich z. B. auch Immunliposomen durch Kopplung von Organ- bzw. gewebespezifischen Antikörpern unter Zusatz von DAC-Chol/DOPE herstellen. Bei vorheriger Inkubation der zu transfizierenden DNA mit Kernproteinen (z. B. HMG-1) läßt sich eine erhöhte Integration und Expression des Fremdgens nach Verkapselung bzw. Assoziation in DAC-Chol/DOPE-Liposomen erreichen. Ein weitere Erhöhung der Aufnahme (Fusion) der DAC-Chol-Liposomen ist möglich, wenn man Fusionsproteine bzw. inaktivierte Viren in die Liposomenmembran rekonstituiert bzw. assoziiert.

Von Vorteil ist ferner, daß man diese Liposomen ohne nennenswerte Toxizitäten bzw. Immunreaktionen über automatische oder nachfüllbare Pumpsysteme zum direkten in vivo-Gentransfer (intratumoral bzw. Organ-spezifisch) verabreichen kann. Mit dieser Methode, die auch für andere Liposomen anwendbar ist, wird eine im Vergleich zu retroviralen bzw. adenoviralen in vivo-Methoden eine weit höhere Transfektionseffektivität erreicht. Damit können Tumorzellen, die sich in unterschiedlichem Maße zu einem bestimmten Zeitpunkt in Proliferation befinden, transfiziert und abgetötet werden.

Die Erfindung soll nachfolgend durch Ausführungsbeispiele näher erläutert werden.

### Ausführungsbeispiele

### 1. Herstellung von 3β(N-(N,N'-Dimethylaminoethan)-carbamoyl)cholesterol (DAC-Chol)

Die symmetrische Form von Dimethylethylendiamin wird nach der Vorschrift von Gao et al (Biochem. Biophys. Res. Comm. 179; 280) mit Chlorformylcholesterol umgesetzt. Das erhaltene ölige Produkt wird einer Säulenchromatographie (Silicagel 60, Laufmittel Trichlormethan/Methanol = 9:1) unterworfen. Das abgetrennte DAC-Chol besitzt nach der dünnschichtchromatografischen Reinigung (Laufmittel: Trichlormethan/Methanol = 65:35) einen Rf-Wert von 0,53-0,57.

Für die weitere Verwendung wird DAC-Chol mit DOPE im Verhältnis 3:2 gemischt.

### 2. Marker- und TNF-alpha-Gentransfer mit kationischen Liposomen in Vergleich zur Calciumphosphat-Präzipitationstechnik (CPPT) in vitro

Der Marker-Gentransfer führt zu einer vierfach höheren Transfektionsrate für kationische Liposomen als mit der CPPT-Methode. Die humanen Zellinien N64 und N31 zeigen beim Vergleich mit den üblichen Transfektionsmethoden (CPPT) eine 4-10fach höhere Transfektionsrate bei Verwendung von DAC-Chol/DOPE-Liposomen. Beim Einsatz von F98 Gioblastoma-Zellen von Ratten werden jedoch nur geringe Unterschiede beobachtet. Interessanterweise ist die Präparation gemäß der Erfindung trotz des aus dem DNA-Anteil resultierenden hohen Gehalt an DAC-Chol/DOPE-Liposomen nicht toxisch, was sowohl anhand von Vitalitätstesten (MTT) als auch an morphologischen Parametern nachgewiesen werden kann.

**Tabelle 1**

| Maximale Transfektionseffizienz (aus 3 Experimenten, in % der transfizierten Zellen (5 x 10⁵, 5 µg DNA (pBAG)) | | | |
|---|---|---|---|
| Transfektions methode | N31-Zellen | N64-Zellen | F98-Zellen |
| DC-Chol | 1.26 | 2.00 | 1.06 |
| Lipofectin | 0.40 | 1.10 | 2.05 |
| Ca₃(PO₄)₂ | 0.12 | 0.52 | 0.76 |

**Tabelle 2**

| hTNF Expression nach Gentransfer von F98 Zellen | |
|---|---|
| Transfektionsmethoden | TNF Aktivität ng/ml von 5 x 10⁴ Zellen, 24h |
| Ca₃(PO₄)₂ | 1,81 ± 0,29 |
| DAC/Chol/DOPE | 1,76 ± 0,3 |
| Lipofectin | 1,76 ± 0,29 |

Die Resultate sind ein Mittel aus 4 Experimenten + Standardabweichung. Der t-Test für unabhängige Proben zeigt keine signifikanten Unterschiede zwischen den Ergebnissen der 3 Techniken.

Die hTNF-Sekretion nach dem Transfer mit den verschiedenen Methoden liegt zwischen 1-2 ng/ml. Die ausgeschiedene TNF-alpha Menge bewirkt eine deutliche Wachstumshemmung von F98-Zellen innerhalb von 5 Tagen. Die Wachstumshemmung wird von morphologischen Änderungen und vom Zelltod begleitet. F98-Zellen, die mit dem leeren (pWG29del3-)Vektor transfiziert wurden, exprimierten keine nachweisbaren Mengen von TNF und erwiesen sich als morphologisch intakt.

### 3. Die Stimulierung der TNF-alpha Expression von DAC-Chol-Liposomen durch Dexamethason

**Tabelle 3**

| Dexamethason-stimulierte Expression von hTNF in transfizierten Zellen | | | | |
|---|---|---|---|---|
| | Nr. des Zellklons Dexamethason (10⁻⁶M/5h) | | TNF Aktivität ng/ml 5 x 10⁵/24 h | x fach |
| Lipofectin | 10 | - | 2.03 | |
| | | ± | 17.89 | 8.9 |
| DAC-Chol/DOPE | 5 | - | 1.12 | |
| | | ± | 19.78 | 17.6 |
| Transfectam | 8 | - | 2.93 | |
| | | ± | 15.34 | 5.3 |

Dexamethason kann die hTNF-Produktion in F98 Zellklonen, gewonnen nach Transfektion mit DAC-Chol-Liposomen, bis zum 18fachen steigern. Ausgehend von den vorhergehenden Ergebnissen werden kationische DAC-Chol-Liposomen für den in vivo-Marker Gentransfer ausgewählt.

### 4. LacZ Gentransfer mit DAC-Chol/DOPE Liposomen in vivo

Nach dem in vivo-Marker Gentransfer mittels DAC-Chol-Liposomen in implantierte Rattentumore (F98, 6 µg LacZ/10 µl DAC-Chol/DOPE) konnte nachgewiesen werden, daß diese Liposomen beim direkten Gentransfer in der Lage waren,, bis zu 3 Zellschichten um die Injektionsebene herum zu transfizieren (gemessen an einer positiven X-Gal-Färbung). In normalem Hirngewebe konnte dagegen keine Färbung durch endogene β-Galactosidase nachgewiesen werden.

## Patentansprüche

1. 3β[N-(N,N'-Dimethylaminoethan)-carbamoyl]cholesterol( DAC-Chol)

2. Verfahren zur Herstellung von DAC-Chol, dadurch gekennzeichnet, daß N,N'-Dimethylethylendiamin und Chlorformylcholesterol in äquimolaren Mengen miteinander umgesetzt werden und das erhaltene Produkt durch Chromatographie gereinigt wird.

3. Verfahren nach Anspruch 2, dadurch gekennzeichnet, daß die Reinigung säulenchromatographisch mit Silicagel und dem Fließmittel Trichlormethan/Methanol 9:1 und nachfolgend dünnschichtchromatographisch mit dem Fließmittel Trichlormethan/Methanol 65:35 erfolgt

4. Verwendung von DAC-Chol für den direkten liposomalen Gentransfer in vivo, ggf. unter Verwendung von Antikörpern, u. a. zur Herstellung von Immunliposomen, von viralen Fusionsproteinen und von Kernproteinen (Nichthistonproteinen) wie HMG-1 durch kontinuierliche oder in gezielten Zeitintervallen wiedeslicht durchgeführte Applikation unter Einsatz von automatischen oder nachfüllbaren Pumpsystemen.

## Claims

1. 3β-[N-(N,N'-dimethylaminoethane)-carbamoyl]-cholesterol (DAC-Chol)

2. Method for preparing DAC-Chol wherein N,N'-dimethylethylene diamine and chloroformyl cholesterol react with each other in equimolar quantities and the product obtained will be purified chromatographically.

3. Method according to claim 2 wherein a purification by means of column chromatography with silica gel and the mobile solvent trichloromethane/methanol 9:1 and subsequently by means of thin layer chromatography with the mobile solvent trichloromethane/methanol 65:35 will be effected.

4. Use of DAC-Chol for the direct liposomal gene transfer *in vivo,* possibly using antibodies, a. o. for the preparation of immune liposomes, viral fusion proteins and nuclear proteins (non-histon proteins) such as HMG-1, by continuous application or repeated application in purposeful intervals of time using automatic or refillable pumping systems.

## Revendications

1. 3β-[N-(N,N'-diméthylaminoéthane)-carbamoyl] -cholestérol (DAC-Chol)

2. Procédé pour la production de DAC-Chol, caractérisé en ce que de la N,N'-diméthylèthylenediamine et du chloroformylcholestérol sont transformés en présence l'un de l'autre en quantités équimolaires et le produit obtenu est purifié par chromatographie.

3. Procédé selon la revendication 2, caractérisé en ce que la purification est effectuée par chromatographie sur colonne avec du gel de silice, en utilisant comme solvant du trichlorométhane et du méthanol dans un rapport de 9:1, puis par chromatographie en couche mince en utilisant comme solvant du trichlorométhane et du méthanol dans un rapport de 65:35.

4. Utilisation du DAC-Chol pour le transfert direct de gènes *in vivo* par des liposomes, en utilisant éventuellement des anticorps, entre autres pour la production d'immunoliposomes, de protéines de fusion virales et de protéines nucléiques (protéines non histones) comme la HMG-1, par application continue ou répétée à des intervalles précis à l'aide de systèmes de pompage automatiques ou rechargeables.
